# EUROPEAN PATENT SPECIFICATION

(11) **EP 2 407 143 B1**
(45) Date of publication and mention of the grant of the patent: **23.01.2013**
(21) Application number: 10173814.4
(22) Date of filing: 24.08.2010
(51) Int. Cl.: A61K 8/02, A61Q 9/04

(54) **Transparent depilatory article**
Transparenter Enthaarungsartikel
Article dépilatoire transparent

(30) Priority: 16.07.2010 US 364828 P
(43) Date of publication of application: 18.01.2012
(73) Proprietor: The Procter & Gamble Company, Cincinnati, OH 45202 (US)
(72) Inventor: Hassan, Cigdem Asli, London, Middlesex SE6 2QA (GB); Robinson, Susan Clare, Twickenham, Middlesex TW2 6RL (GB); Smith, Paul James, Whitton, Middlesex TW2 6EB (GB); Fletcher, Jamie Anthony, Luston, Hereford and Worcester HR6 0EB (GB); Passi, Rajeev Kumar, West Chester, OH 45069 (US); Mitra, Shekhar, Cincinnati, OH 45243 (US); Sagel, Paul Albert, Maineville, OH 45039 (US)
(74) Representative: Simpson, Kirsty Mairi

(56) References cited:
- WO-A1-03/075812
- GB-A- 1 291 377
- US-A1- 2002 146 380
- DATABASE Gnpd [Online] mintel; June 2004 (2004-06), anonymous: "Hair removal Strips", XP002617254, Database accession no. 276929

## Description

### FIELD OF THE INVENTION

The present invention relates to depilatory articles comprising a chemically active depilatory composition disposed on a substrate.

### BACKGROUND OF THE INVENTION

Depilatory compositions used to remove unwanted hair by chemical activity are known. Such compositions may comprise thioglycolate salts to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion. By disposing the depilatory composition on a substrate one may overcome or mitigate such disadvantages to an extent. Substrate-based depilatory products are known from JP63073910A, US2006002878, JP6135826A, JP11012123A and JP62230711A.

While addressing some of the handling problems of creams and lotions by removing the need for an application implement, known substrate-based depilatory compositions do not address the problem of ensuring accurate positioning of the depilatory article, nor the resultant risks of irritation or ineffective depilation that may be associated with uncontrolled application. Specifically, applicants have found that being unable to see through the depilatory article allows it to be misplaced upon the surface of the body, therefore leading to depilatory composition being applied to areas with no unwanted hair, and/or depilatory composition not being applied to areas with unwanted hair. This inaccurate application may lead to unnecessary and unpleasant irritation in some areas and insufficient depilatory action in others. Simultaneously, an inability to see the depilatory composition acting upon the hairs during usage may lead to leaving the depilatory article in place for an excessive or insufficient period of time in spite of any instructions included with the product. Excessive exposure to the depilatory composition may also lead to skin irritation, and insufficient exposure may lead to inefficient hair removal. There exists a need, therefore, for an effective substrate-based depilatory article that facilitates the user being able to accurately place the depilatory article and judge when the chemical depilation process is sufficiently complete.

### SUMMARY OF THE INVENTION

According to a first aspect of the invention, the applicants have found that a depilatory article according to claim 1, meets the aforementioned need by providing a depilatory article which may be accurately placed by the user, and enables him or her to assess when the chemical depilation process has sufficiently finished.

According to a second aspect of the invention, a cosmetic method of removing hair from the skin is provided, comprising the steps of: applying a depilatory article according to the first aspect of the invention to a surface of skin, preferably human skin, leaving said depilatory article in contact with the skin for a period of greater than 1 minute, preferably 2 to 10 minutes, more preferably 3 to 8 minutes, removing said depilatory article from the surface of the skin, and preferably rubbing, scraping, rinsing or wiping the surface of the skin in the area to which the depilatory article was applied.

According to a third aspect of the invention, a depilatory kit is also provided, comprising: a depilatory article according to the first aspect of the invention, optionally at least one of a pre-treatment depilatory composition, a post-treatment depilatory composition and/or a tool to assist removal of hair and/or depilatory composition after use, and packaging for said depilatory kit.

### BRIEF DESCRIPTION OF THE DRAWINGS

Fig.1. is a plan view of a depilatory article of the present invention.
Fig.2. is a side view of a depilatory article of the present invention.
Fig.3. is a side view of a depilatory article of the present invention applied to keratinous tissue.
Fig.4. is a plan view of a depilatory article of the present invention applied to keratinous tissue.

### DETAILED DESCRIPTION OF THE INVENTION

Unless otherwise specified, all parts, quantities, fractions, ratios, and percentages herein are indicated by weight, and all measurements are made at 25°C.

As used herein, the term "transparent" refers to materials, articles, substrates and/or compositions through which it is possible to see with the naked eye including, but not limited to, materials, articles, substrates and/or compositions through which the skin and/or hair may be identified in-use. Advantageously, "transparent" includes materials, articles, substrates and/or compositions with a total transmittance of at least 60%, preferably at least 70%, more preferably at least 80%, even more preferably at least 85% and even more preferably still at least 90%. The total transmittance of a material, article, substrate and/or composition may be measured at 23°C according to ASTM D1003-07 on the measuring apparatus Haze Gard Plus available from the Byk Gardner Company using a CIE Illuminate C light source. The total transmittance is the ratio of total transmitted light to incident light and is expressed as a percentage, being for example the average of 9 measurements made as 3 sets of 3 measurements upon each of 3 independent and freshly prepared samples. Measurements of samples comprising a composition may use a path length within the composition of 0.30 mm (300 microns).

As used herein the term "non-reflective" refers to materials, articles, substrates and/or compositions that do not scatter sufficient incident light back to the viewer to prevent them identifying the skin and/or hair through the materials, articles, substrates and/or compositions Advantageously, "non-reflective" includes articles and/or compositions with a reflectance less than 40%, preferably less than 30%, more preferably less than 20%, even more preferably less than 10% and even more preferably still less than 5%. The reflectance of a material, article substrate and/or composition may be measured using a Spectroflash 600 spectrophotometer available from Datacolor International operating over a wavelength range of 400-700 nm by firstly measuring the light reflected by a black Sheen Hiding Power Card (available from Sheen Instruments Ltd. (Ref. 301-AU)) mounted perpendicular to the incident beam to obtain a reference reflectance, then secondly mounting the sample against the black Sheen Hiding Power Card and measuring the light reflected from the combination of the black Sheen Hiding Power Card and the sample to obtain a combined reflectance. The reference reflectance is subtracted from the combined reflectance to obtain the reflectance of the sample, i.e. the reflectance of the material, article, substrate and/or composition, which may be reported as an average value over the wavelength range used. The reflectance is the ratio of reflected light to incident light and is expressed as a percentage, being for example the average of 9 measurements made as 3 sets of 3 measurements upon each of 3 independent and freshly prepared samples. Measurements of samples comprising a composition may use a path length within the composition of 0.30 mm (300 microns).

As used herein the term "non-hazy" refers to materials, articles, substrates and/or compositions that do not internally scatter sufficient incident light to prevent the viewer from identifying the skin and/or hair through the materials, articles, substrates and/or compositions. Advantageously, "non-hazy" includes materials, articles, substrates and/or compositions with a haze less than 70%, preferably less than 50%, more preferably less than 40%, even more preferably less than 30% and even more preferably still less than 20%. The haze of a material, article, substrate and/or composition may be measured according to ASTM D1003-07 on the measuring apparatus Haze Gard Plus available from the Byk Gardner company using a CIE Illuminate C light source at 23°C. The haze is the percentage of transmitted light that deviates from the incident beam by more than 2.5° on the average and is expressed as a percentage, being for example the average of 9 measurements made as 3 sets of 3 measurements upon each of 3 independent and freshly prepared samples. Measurements of samples comprising a composition may use a path length within the composition of 0.30 mm (300 microns).

As used herein the term "colloid-forming" includes chemical species that are able to form stable (i.e. no visible separation after 3 months in a sealed container stored at 40°C in a 50% relative humidity atmosphere), aqueous solid-in-liquid colloidal systems, including nano-colloidal systems.

As used herein, the term "buffering base" refers to a base capable of opposing pH changes by means of chemical or physical (solubility) processes and thereby limiting the pH to less than or equal to 13.

As used herein, the term "water impermeable" includes materials, substrates, articles, compositions and/or objects through which water in its liquid state does not pass.

Depilatory articles of the present invention comprise a substrate, to facilitate application of the depilatory composition to keratinous tissue and prevent a messy usage experience. The substrate preferably comprises a coated or impregnated region comprising depilatory composition. At least a portion of the substrate is transparent, alternatively the whole of the substrate is transparent and alternatively again the substrate comprises at least one region (which may be a partial or complete border region around the edge of the substrate) that is not transparent and at least one second region that is transparent. Applicants have found that selecting a substrate with at least a portion that is transparent enables the user to more accurately position the depilatory article against the surface of the body, by allowing the user to see the surface of the body and correctly place the depilatory article. Additionally, unwanted hairs selected for treatment become pressed against the substrate by the application of the depilatory article to a surface of the body, enabling the user to see them and consequently to visually assess progress being made by the depilatory activity rather than relying on estimated timings alone, as the hairs curl and crinkle during exposure to the depilatory composition. This aids the user in improving depilatory efficacy while reducing the risk of skin irritation. A depilatory article in which the whole of the substrate is transparent enables the user to see the whole area of the body to which the depilatory article is being applied to enable accurate positioning, while having regions of transparent and non-transparent substrate may provide indications of suitable sites for holding the article, guides to aid placement of the article upon the body, or supports that increase the rigidity of the depilatory article to aid handling.

The depilatory article may comprise an indicium, marking, indicia or markings to guide or aid the user in holding, positioning, applying, using or removing the depilatory article, such as by showing a suitable site at which to hold the article, to enable alignment of the article with a portion of the surface of the body, to provide safety information or guidance on applying or removing the depilatory article or the duration for which to leave the depilatory article applied to the surface of the body. Non-limiting examples of indicia or markings include a mark to enable alignment of a depilatory article with the corner of a mouth or the centre of the armpit, or designs upon a region of the substrate that is free of depilatory composition to indicate to the user to hold the article in that position. Separate indicia relating to markings upon the depilatory article may also be employed.

Advantageously, at least 30% of the surface area of the substrate is transparent, preferably from 40% to 99%, more preferably from 50% to 95%, even more preferably from 60% to 90% and even more preferably still from 70% to 85%. Within the preferred ranges, sufficient area of the substrate is transparent to enable the user to accurately apply the depilatory article, while also providing an adequate area upon which to include holding, positional, application, usage or safety indicia. In a particularly preferred embodiment, the substrate of the depilatory article is transparent over at least a portion of, and more preferably the entirety of, the coated or impregnated region. In a further preferred embodiment, any transparent portion of the substrate of the depilatory article is transparent when observed from either side.

It may also be advantageous to use a coloured substrate in order to enable the user to readily distinguish unwanted hair during use of the depilatory article. Typical hair colours are white, grey, black, brown, red (ginger) and blonde. Accordingly, in a preferred embodiment, the substrate of the depilatory article is at least partially coloured and may be coloured throughout, more preferably in a colour not matching a typical hair colour or a colour complementary to typical hair colours. Advantageously, the substrate is colourless, coloured green, blue, purple (violet) or pink, more preferably the substrate is colourless, coloured green or blue and even more preferably the substrate is colourless or coloured blue. In a particularly preferred embodiment, the coloured portion extends over at least the coated or impregnated region of the substrate.

Light reflected from the surface of the depilatory article (especially by specular reflection) may interfere with the user's view of the surface of the body and unwanted hair beneath the depilatory article during application or in-use. Accordingly, in a preferred embodiment, at least a portion of the surface of one side of the substrate (especially the surface facing away from the surface of the body in use) is non-reflective. Preferably, at least the coated or impregnated region of at least one side of the substrate is non-reflective and even more preferably the whole of at least one side of the substrate is non-reflective. In a further advantageous embodiment, any region of the substrate that is non-reflective on one side is additionally non-reflective on the opposing side. Accordingly, the surface or surfaces of the substrate may not have a glossy finish, but may instead be matt or dull in order to reduce specular reflection.

Light scattering within the substrate may also interfere with the user's view of the surface of the body and unwanted hair beneath the depilatory article during application or in-use. Accordingly, it is advantageous to use a non-hazy substrate. Accordingly, in a preferred embodiment, at least a portion of the surface of one side of the substrate (especially the surface facing away from the surface of the body in use) is non-hazy. Preferably, at least the coated or impregnated region of at least one side of the substrate is non-hazy and even more preferably the whole of at least one side of the substrate is non-hazy. In a further advantageous embodiment, any region of the substrate that is non-hazy when observed from one side is additionally non-hazy when observed from the opposing side. In another preferred embodiment, at least a portion of the surface of one side of the substrate (especially the surface facing away from the surface of the body in use) is both non-reflective and non-hazy. Preferably, at least the coated or impregnated region of at least one side of the substrate is both non-hazy and non-reflective and even more preferably the whole of at least one side of the substrate is both non-hazy and non-reflective. In a further advantageous embodiment, any region of the substrate that is both non-reflective and non-hazy from one side is additionally both non-reflective and non-hazy from the opposing side.

In order to improve the transparency and reduce the haziness of the substrate of the depilatory article, it may be advantageous to limit the quantity of particulate materials included therein in order to reduce the extent to which light scatters inside the substrate. Accordingly, in a preferred embodiment, the substrate may comprise particulate materials at a level of no more than 20%, more preferably less than 10%, even more preferably less than 5% and even more preferably still less than 3%. Non-limiting examples of particulate materials include titanium dioxide, marine pigments, calcium carbonate and calcium chloride.

The substrate may comprise a textured or, alternatively, micro-structured surface on at least a portion of one side. Surface texturing or micro-structuring increases the effective surface area of the substrate and thus improves adhesion of the depilatory composition to said substrate, facilitating an easy removal of the depilatory article by peeling it off the skin, or increases the grip of the surface, thus improving handleability and/or aesthetics. The textured structures may comprise dimples; lines or curvilinear embossments. A textured surface may be formed on the substrate by any appropriate technique, including embossment calendars and casting. However, texturing the surface of the substrate may increase light scatter thereon. Accordingly, in an alternative embodiment, at least a portion of at least one side of the substrate is untextured and/or substantially smooth, alternatively again at least a portion of the coated or impregnated region is untextured and/or substantially smooth, in a further alternative at least the entirety of the coated or impregnated region of the substrate is untextured and/or substantially smooth and in another alternative the whole of the side of the substrate is untextured and/or substantially smooth. In another alternative embodiment, any region of the substrate that is untextured and/or substantially smooth on one side is additionally untextured and/or substantially smooth on the opposing side.

Depilatory articles according to the present invention further comprise a depilatory composition comprising a keratin reducing agent and being in physical contact with the substrate, preferably forming a coated or impregnated region of the substrate. Preferably, the depilatory composition is in physical contact with a transparent portion of the substrate. Light absorbed by the depilatory composition may interfere with the user's view of the surface of the body and unwanted hair. Accordingly, in an advantageous embodiment, the depilatory composition is also transparent. Light reflected from the depilatory article may also interfere with the user's view of the surface of the body and unwanted hair beneath the depilatory article during application or in-use. Accordingly, in a preferred embodiment, the depilatory composition is non-reflective. Light scattering within the depilatory article may also interfere with the user's view of the surface of the body and unwanted hair beneath the depilatory article during application or in-use. Accordingly, in a preferred embodiment, the depilatory composition is non-hazy. In further preferred embodiments, the depilatory composition is transparent and non-reflective, transparent and non-hazy, non-reflective and non-hazy, or transparent, non-reflective and non-hazy.

Applicants have found that combining a transparent depilatory composition with a transparent substrate improves the user's ability to accurately place the depilatory article and the user's capacity to visibly see the effects of the depilatory composition by making the hairs and surface of the body more visible to the user both during application and use. In a preferred embodiment, the transparent depilatory composition is in contact with a transparent portion of the substrate. The combination of the substrate and the depilatory composition is transparent in at least a portion of the coated or impregnated region, and preferably the combination of the substrate and the depilatory composition is transparent in the whole of the coated or impregnated region. In yet another advantageous embodiment the combination of the substrate and the depilatory composition is non-reflective in at least a portion of the coated or impregnated region, and preferably the combination of the substrate and the depilatory composition is non-reflective in the whole of the coated or impregnated region. In a further advantageous embodiment the combination of the substrate and the depilatory composition is non-hazy in at least a portion of the coated or impregnated region, and preferably the combination of the substrate and the depilatory composition is non-hazy in the whole of the coated or impregnated region. In an additional advantageous embodiment, at least a portion of the coated or impregnated region (preferably the whole of the coated or impregnated region) is transparent and non-reflective, transparent and non-hazy, non-reflective and non-hazy, or transparent, non-reflective and non-hazy.

In order to improve the transparency and reduce the haziness of the depilatory composition of the depilatory article, it may be advantageous to limit the quantity of colloid-forming materials included therein. Accordingly, in a preferred embodiment, the depilatory composition may comprise colloid-forming materials at a level of no more than 20%, more preferably less than 10%, even more preferably less than 6% and even more preferably still no more than 4%. Non-limiting examples of colloid-forming materials include a fatty or oil phase dispersed in a continuous aqueous phase and particulate materials such as titanium dioxide or calcium carbonate. In an alternative preferred embodiment, the depilatory composition is not a colloid or alternatively not an emulsion.

In a further advantageous embodiment, the depilatory composition has a mean thickness of from 0.01 mm to 3 mm, more preferably from 0.01 mm to 1.5 mm, even more preferably from 0.05 mm to 0.8 mm, and even more preferably still from 0.05 mm to 0.5 mm. Within the preferred thickness ranges, the applicants have found that depilatory efficacy may be maintained, and handleability promoted to enable accurate placement of the depilatory article. Simultaneously, the hairs and surface of the body may rest close enough to the substrate of the depilatory article once applied in order to be seen more clearly by the user. When a preferred transparent depilatory composition is used, limiting the thickness of the composition is also advantageous, as excessive quantities of depilatory composition may occlude viewing of the hair and surface of the body through the depilatory article.

The rigidity of the substrate also promotes desirable handleability and conformability attributes in a depilatory article, notably to enable accurate placement of the article. In particular, the article collapsing under gravity or folding is avoided, which is especially undesirable if different areas of the depilatory composition are able to readily come into contact with each other, while maintaining the capability for the substrate to conform to the surface to which it is applied without folding or crinkling, in order to further improve depilatory efficiency. Accordingly, the substrate is preferably conformable to the skin and unwanted hair without permanently deforming during use, as this may also result in problems for the user during application. In a preferred embodiment, the rigidity is substantially constant and does not change during the lifetime of a product. The rigidity of a substrate is a function of substrate thickness and inherent modulus of elasticity. Different materials have different moduli of elasticity. Based upon the material or materials that the substrate comprises, a substrate thickness may be selected that enables the desired rigidity of the substrate to be achieved.

Rigidity can be readily measured using the American Standard Test Method (ASTM) D2923-06 on a Handle-O-Meter, model #211-300, available from Thwing-Albert Instrument Co. of Philadelphia, Pa. The measured value (in grams) of the sample read directly from the meter is normalized to determine the rigidity of the material itself by expressing the measured value as grams per centimetre of sample width. Samples of substrate were prepared as 10.16 cm (4 inch) by 10.16 cm (4 inch) test specimens with edges parallel to the machine direction and transverse direction for substrates with directionality. Three rigidity measurements are determined on the same side of fresh test specimens orientated in the same substrate direction. A further three rigidity measurements are taken on the same side of fresh test specimens oriented at 90° to the first orientation. These six measurements are repeated on the opposite side to the first six measurements, on fresh test samples. The 12 rigidity measurements are then averaged and reported to 0.01 g/cm.

The substrate has a thickness of from 50 µm to 15 µm, more preferably from 40 µm to 16 µm and even more preferably from 30 µm to 17 µm. Additionally, limiting the thickness of the substrate further promotes the visibility of the hair and surface of the body when the article is applied.

The depilatory composition should be suitable for being placed in contact with a user's skin (and unwanted hair). The depilatory composition is aqueous. The concentration of water in the depilatory composition is preferably at least 40%, more preferably from 50% to 98%, even more preferably from 60% to 95% and even more preferably still from 70% to 90%, by weight of the depilatory composition. As water is a transparent material, this high water level helps to improve the transparency of the depilatory composition and improve the overall skin mildness of the depilatory composition by being dilute, and to keep the system more robust to pH changes, which may result in skin irritation.

The rheological properties of the depilatory composition may also lead to improved performance in use. In particular, the yield point describes the resistance of the depilatory composition to deformation under environmental stress. If the yield point is too high, then the depilatory composition may not deform sufficiently, with hair fibres unable to enter the depilatory composition effectively upon application, resulting in less desirable depilatory effectiveness. If the yield point is too low, however, then the depilatory composition may flow during storage, transport or use leading to areas of differing transparency, and is not cleanly removed from the skin upon removal of the depilatory article, thus requiring the inconvenience of additional wiping and risking irritation to the user. Accordingly the depilatory composition preferably has a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

Advantageously, the depilatory composition displays an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the depilatory composition exceeds its viscous modulus at a low frequency of applied stress. This indicates that the depilatory composition is behaving in a solid-like manner at rest and is of particular benefit when the depilatory composition is interposed between two substrates, for example a substrate and a protective release layer.

In another preferred embodiment, the depilatory composition displays a high degree of shear thinning behaviour enabling the effective coating of target hairs during application and improve depilatory efficacy. Accordingly, at a low shear rate of 0.1 s⁻¹, the dynamic viscosity of the depilatory composition is preferably 1000 Pa.s to 10000 Pa.s measured at a temperature of 25°C, whereas at a high shear rate of 1000 s⁻¹, the dynamic viscosity of the depilatory composition is preferably 0.1 Pa.s to 1 Pa.s, measured at a temperature of 25°C.

The substrate may be water permeable or water impermeable. The substrate may comprise any suitable material such as fibrous materials, papers, fabrics, non-wovens, plastics, amorphous solids, crystalline solids, rubbers, latex, thermoplastic elastomers, cellular foams (open cell), composites, laminates and mixtures thereof. Preferably, the substrate is water impermeable. Using a water impermeable substrate prevents water loss from the depilatory composition while the depilatory composition is in contact with the keratinous tissue and thus prevents the depilatory composition from drying out. Water loss from the depilatory composition lowers the water concentration, thus increasing the concentration of active ingredients and bases present. This could result in a reduction of transparency and/or irritation to the skin, which applicants wish to avoid. The substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The substrate may comprise a single polymer or mixtures of polymers or copolymers. Preferably the substrate comprises a plastic sheet, more preferably a polyolefin, even more preferably a polyethylene and even more preferably still high density polyethylene. In an advantageous embodiment, the depilatory composition is disposed upon or within the water impermeable material, preferably plastic sheet, more preferably polyolefin, even more preferably polyethylene and even more preferably still high density polyethylene. In this advantageous embodiment, there is preferably no layer of water permeable material between the depilatory composition and the water impermeable material. In a preferred embodiment, the water impermeable material forms a water impermeable layer. The substrate may be layered or a laminate comprising at least one water impermeable material with combinations of other substrates or multiple laminations or layers, including non-wovens; paper; or topical coatings (e.g. surfactants; printing); open cell foams or substrates described herein above.

The substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions and topical surface applications.

A layer of depilatory composition can be applied to the substrate through any known technique of applying viscous fluids to or into substrates, including, for example, extrusion, casting (e.g., reverse roll, knife-over roll, slot die, Gravure roll), spraying, knife blade coating, and zone coating. Such techniques may be modified to alter the quantity of depilatory composition disposed on the substrate. Advantageously, the depilatory composition covers less than the entire surface of the substrate to facilitate handling. The substrate may comprise at least one region with two orthogonal dimensions each of a length greater than 1 cm, preferably greater than 1.5 cm and more preferably greater than 2 cm upon which no depilatory composition is disposed.

Preferably, the depilatory composition is disposed upon the substrate in an amount per unit area of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm², wherein the unit area refers to the coated or impregnated region of the substrate and not including any untreated surface of the substrate. Additionally, the area used to calculate the amount of depilatory composition disposed upon the substrate is calculated ignores any surface texturing or micro-structuring. Alternatively, the mean thickness of the depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

The depilatory composition comprises a keratin reducing agent to weaken and/or break strands of unwanted hair. Non-limiting examples of suitable keratin reducing agents include: sulphide salts such as Li₂S, Na₂S, K₂S, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition. Advantageously, the depilatory composition may comprise at least one thioglycolate salt or thioglycollic acid acting as a hair removal agent when the depilatory composition is applied to unwanted hair. Preferably, the depilatory composition comprises sodium, potassium, lithium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof. In a preferred embodiment, the concentration of the conjugate acid of the thioglycolate salt, (which includes all species in the deprotonation equilibrium system) is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the depilatory composition. Advantageously, the quantity of monovalent cations (or monovalent metal cations in the preferred embodiment above) per unit area of the depilatory article (not including uncoated or unimpregnated regions thereof) is less than 5.10 × 10⁻⁴ mol/cm², preferably less than 3 × 10⁻⁴ mol/cm⁻², more preferably from 1 × 10⁻⁹ mol/cm² to 1.5 × 10⁻⁴ mol/cm², even more preferably from 2.50 × 10⁻⁸ mol/cm² to 6.65 × 10⁻⁵ mol/cm² and even more preferably still from 6 × 10⁻⁷ mol/cm² to 4.5 × 10⁻⁵ mol/cm². The amount of monovalent cations may be calculated based upon the ingredients within the depilatory composition, or measured (for example using Elemental Analysis by Inductively Coupled Plasma) and expressed per unit area of the coated or impregnated region. The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula if monovalent ion containing thioglycolate salts or bases are used. Accordingly, in an advantageous embodiment, the depilatory composition comprises a divalent cation, preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide.

In a preferred embodiment, the base is present at a concentration of from 0.1 % to 10.0%, more preferably from 0.5% to 8.0% and even more preferably from 1.0% to 5.0%, by weight of the depilatory composition.

The depilatory composition may optionally comprise a further thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. The thickening agent may be present at a level of from about 0.01% to about 20%, preferably from about 0.1% to about 10%, more preferably from about 0.3% to about 5%, and even more preferably from about 0.5% to about 3%, by weight of the depilatory composition.

Advantageously, the depilatory composition comprises carrageenan. The carrageenan is preferably present in an amount of from 0.1% to 10%, more preferably from 0.5% to 8%, even more preferably from 1% to 5% and even more preferably still from 2% to 4% by weight of the aqueous depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001 % to about 10%, more preferably from about 0.01 % to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in hair removal compositions, particularly dyes; pigments; anionic, cationic, non-ionic and/or amphoteric or zwitterionic surfactants, polymers; dispersing agents; solvents; lubricants; fragrances; preservatives; chelants, proteins and derivatives thereof, plant materials (e.g. aloe, chamomile and henna extracts); film-forming agents; film forming promoters and mixtures thereof.

Depilatory articles of the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the substrate of the depilatory article is substantially planar.

Depilatory articles of the present invention may comprise at least two finger-tabs being substantially free of depilatory composition and positioned on substantially opposing sides of the coated or impregnated region. These finger tabs enable a user to apply tension to the coated or impregnated region of the substrate. Applicants have found that applying tension across the coated or impregnated region of the depilatory article creates an effect of temporarily causing the coated or impregnated region to exhibit an apparent increased rigidity, enabling the user to accurately position the depilatory article, and hence depilatory composition on to the desired region of the body. Tensioning the coated or impregnated region may be achieved in a number of ways, non-limiting examples of which include holding the depilatory article either side of the coated or impregnated region, for example with the hands or a tool, so as to apply tension between the areas being held. Alternatively, depilatory articles of the present invention may comprise at least one finger-tab being substantially free of depilatory composition and positioned to allow the weight of the article to tension the coated or impregnated region when being held by the finger-tab.

In a preferred embodiment, at least one finger tab extends from the perimeter of the coated or impregnated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated or impregnated region by a minimum of 1 cm, preferably from 1.5 cm to 5 cm, more preferably from 2 cm to 4 cm and even more preferably from 2.5 cm to 3.5 cm, in order to aid handling of the depilatory article.

Depilatory articles of the present invention may comprise a protective release layer removably attached to the depilatory composition, preferably on a surface of the depilatory composition substantially opposing that which is in contact with the substrate. The protective release layer may comprise materials including polymer resins such as a polyolefins e.g. polypropylene (including biaxially oriented polypropylene (BOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective release layer may comprise one or more laminations, combinations of multiple layers and/or indications (which may include instructions and illustrations) relating to at least one aspect of the usage of the depilatory article. In an advantageous the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated or impregnated region of the substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated or impregnated region of the substrate to provide a removal tab.

In a preferred embodiment, the depilatory articles of the present invention are packaged to prevent water loss and/or oxygen permeation. Alternatively, the depilatory articles of the present invention are packaged in water impermeable packaging. Examples of suitable packaging materials include films of EVOH; PP; PE; Nylon; foil laminates (including metalized PET; BOPP and PE), mixtures thereof, laminates thereof or multi-laminates thereof. More preferably, the packaging comprises an inert gas and even more preferably the inert gas comprises at least one of nitrogen, argon or carbon dioxide. Alternatively, the packaging comprises a partial vacuum.

A second aspect being a method of removing hair from the skin is also provided by the present invention, comprising the steps of:
(a) applying a depilatory article according to the present invention to the surface of the skin, preferably mammalian and more preferably human skin,
(b) leaving said depilatory article in contact with the skin for a period of at least 1 minute, preferably 2 to 10 minutes, more preferably 3 to 8 minutes
(c) removing said depilatory article from the surface of the skin, and
(d) preferably rubbing, scraping, rinsing or wiping the surface of the skin in the area to which the depilatory article was applied.

Advantageously, the method of removing hair from the skin further comprises the step of tensioning the coated or impregnated region of the depilatory article prior to applying it to the skin.

The same means used to apply tension to the coated or impregnated region may be used to ensure that the depilatory article is applied to the surface of the body such that the coated or impregnated region is applied under tension to the unwanted hair in order to maintain the improved handling characteristics described above. In a preferred embodiment, the tension is kept substantially constant during application of the depilatory article. A flexible nature to the substrate allows the substrate to conform to the surface of the body to offer improved contact between the depilatory composition and the unwanted hair. In a preferred embodiment, the tension may be at least partially, more preferably substantially completely released from the coated or impregnated region after applying the depilatory article to the skin in order to improve the conformability of the depilatory article.

A third aspect being a depilatory kit is also provided by the present invention, which comprises at least one depilatory article of the present invention, packaging for said depilatory article(s), and at least one of a third component selected from:
a) a pre-treatment skin care composition which may comprise ingredients to promote skin conditioning (e.g. emollients), hair hydration or provide a skin barrier (e.g. hydrophobic materials) and intended for use prior to applying the depilatory article.
b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.
c) a tool to assist in the removal of hair and/or depilatory composition from the skin.
d) indications (which may include instructions and/or illustrations) relating to at least one aspect of usage of the depilatory article or another component of the kit.

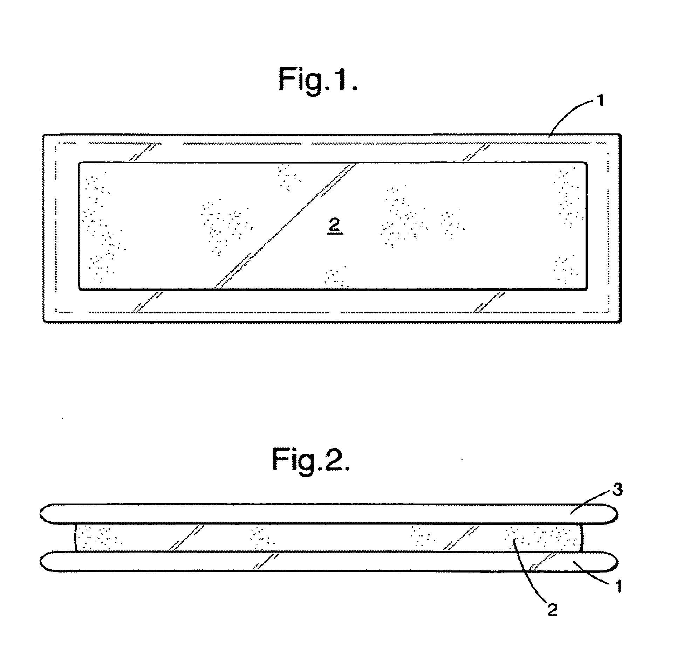

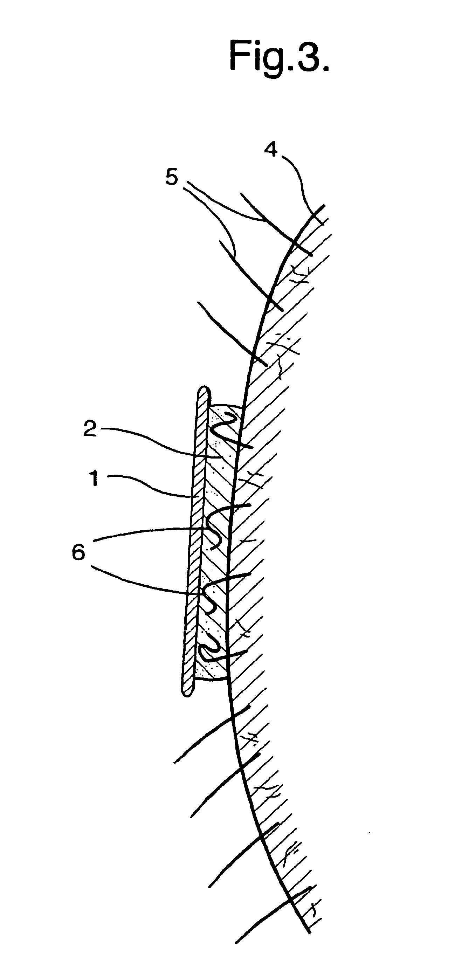

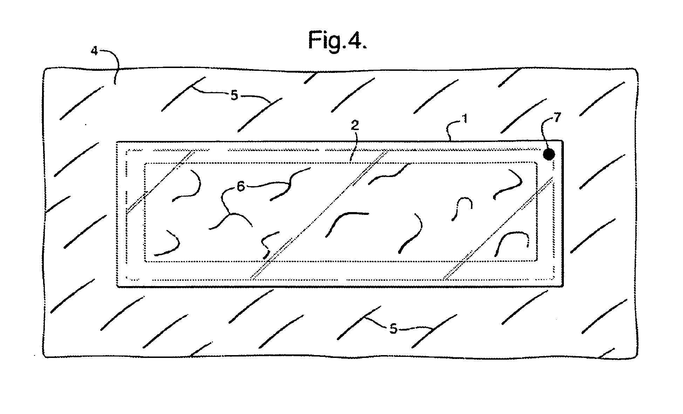

Reference is made to the figures, which disclose a non-limiting embodiment of the invention. Fig.1 depicts a plan view of a depilatory article of the present invention, comprising a transparent substrate (1) and a depilatory composition (2). Fig. 2 depicts a side view of a depilatory article of the present invention, further comprising a protective release layer (3). Fig.3 depicts a side view of a depilatory article of the present invention in use, i.e. applied to keratinous tissue which comprises the skin (4), hair strands (5) outside the depilatory composition (2) and hair strands (6) within the depilatory composition (2). Fig.4 depicts a plan view of a depilatory article of the present invention in use, i.e. applied to keratinous tissue which comprises a marking to aid usage (7), the skin (4), hair strands (5) outside the depilatory composition (2) and hair strands (6) within the depilatory composition (2).

### Example

The following example further describes and demonstrates one embodiment within the scope of the present invention. The example is given solely for the purpose of illustration and is not to be construed as a limitation of the present invention, as many variations thereof are possible.

| Formulation Ingredients | % w/w |
|---|---|
| DI water | 87.05 |
| Carrageenan¹ | 2.60 |
| Sodium Silicate (42% w/w in water) (Cognis 60)² | 1.60 |
| Calcium Hydroxide³ | 3.25 |
| Calcium Thioglycolate Trihydrate⁴ | 5.5 |

| | |
|---|---|
| 1 Carrageenan CI-123 available from CPKelco 2 Sodium Silicate (Cognis 60) available from Cognis 3 Calcium Hydroxide Reag. Ph. Eur. puriss. p.a. available from Sigma-Aldrich Co. 4 Calcium Thioglycolate Trihydrate 99.8% available from BRUNO BOCK Chemische Fabrik GmbH & Co. | |

A 400 ml speed mixer plastic pot was sanitized and DI water weighed in directly. The Calcium Hydroxide was added with mixing and the sodium silicate added slowly with mixing. The carrageenan was then added slowly, alternating from high to low mixing speeds as needed to hydrate the carrageenan. The calcium thioglycolate was slowly added with mixing ensuring uniform distribution. The mixture was then milled using an IKA T50 milling machine operating at a speed of 5200rpm. The total transmittance of a 0.30 mm (300 microns) thick sample of this depilatory composition was 93.5%.

The above formulation was disposed to a thickness of 0.30 mm (300 microns), on a transparent cast polymer blend film (85% transparent HDPE, 15% transparent LLDPE manufactured on a Merritt-Davis casting line with LBI 85% M6030 and Exxon Mobil 15% LD2001); 23 microns in thickness, cut to be 6 cm in length and 6 cm in width and free of opacifiers or opaque printing (total transmittance of 94.1 % and haze of 18.8%) using a stencil (0.30 mm thick) and wiper blade. The cast HDPE 85% LLDPE 15% polymer blend film has a rigidity of 0.47 g/cm as measured on a Handle-o-Meter according to the American Standard Test Method (ASTM) D2923-06. The total transmittance through the substrate and depilatory composition was 88.0%.

## Claims

1. A depilatory article comprising:
(a) a substrate with a mean thickness of from 50 µm to 15 µm (1); and
(b) an aqueous depilatory composition (2) comprising a keratin reducing agent and being in physical contact with the substrate, preferably forming a coated or impregnated region of the substrate;
wherein at least a portion of the substrate is transparent and wherein at least a portion of the coated or impregnated region of the substrate is transparent.

2. A depilatory article according to claim 1 wherein the whole of the coated or impregnated region of the substrate is transparent.

3. A depilatory article according to either preceding claim, wherein the depilatory composition is transparent.

4. A depilatory article according to any preceding claim, wherein the substrate, depilatory composition or both further comprises at least one marking to guide or aid the user in holding, positioning, applying, using or removing the depilatory article.

5. A depilatory article according to any preceding claim, wherein the substrate is nonreflective.

6. A depilatory article according to any preceding claim, wherein the substrate is at least partially colourless, blue, green, pink or purple, preferably colourless, blue or green and more preferably colourless or blue.

7. A depilatory article according to any preceding claim, wherein the combination of the substrate and the depilatory composition is transparent in at least a portion of the coated or impregnated region, preferably wherein the combination of the substrate and the depilatory composition is transparent in the whole of the coated or impregnated region.

8. A depilatory article according to any preceding claim wherein the depilatory composition has a mean thickness from 0.01 mm to 3 mm, preferably from 0.01 mm to 1.5 mm, more preferably from 0.05 mm to 0.8 mm, and even more preferably from 0.05 mm to 0.5 mm.

9. A depilatory article according to any preceding claim, wherein the substrate has a mean thickness of from 40 µm to 16 µm and preferably from 30 µm to 17 µm.

10. A depilatory article according to any preceding claim, wherein the substrate is flexible, preferably possesses a rigidity in the range of from 5.00 g/cm to 0.08 g/cm, more preferably from 3.00 g/cm to 0.08 g/cm, even more preferably from 1.80 g/cm to 0.10 g/cm, even more preferably still from 0.80 g/cm to 0.15 g/cm and yet more preferably from 0.60 g/cm to 0.25 g/cm.

11. A depilatory article according to any preceding claim, wherein the depilatory composition comprises water in an amount of at least 40%, preferably from 50% to 98%, more preferably from 60% to 95% and even more preferably from 70% to 90%, by weight of the depilatory composition.

12. A depilatory article according to any preceding claim, wherein the depilatory composition has a yield point from 10 to 2000 Pa, preferably from 30 to 1200 Pa, more preferably from 45 to 500 Pa and even more preferably from 60 to 300 Pa measured via a stress controlled amplitude sweep at a frequency of 1Hz and a temperature of 25°C.

13. A depilatory article according to any preceding claim, wherein the substrate is water impermeable, preferably wherein the substrate comprises a water impermeable polymeric material, more preferably comprises at least one polymer resin, and even more preferably comprises a polyolefin.

14. A method of removing hair from the skin, comprising the steps of:
(a) applying a depilatory article according to any preceding claim to the surface of the skin, preferably mammalian and more preferably human skin,
(b) leaving said depilatory article in contact with the skin for a period of greater than 1 minute, preferably 2 to 10 minutes, more preferably 3 to 8 minutes
(c) removing said depilatory article from the surface of the skin, and
(d) preferably rubbing, scraping or wiping the surface of the skin in the area to which the depilatory article was applied

15. A depilatory kit, comprising:
(a) A depilatory article according to any of claims 1-13,
(b) At least one of a pre-treatment skin care composition, a post-treatment skin care composition and/or a tool to assist removal of hair and/or depilatory composition after use, and
(c) Packaging for said depilatory kit.

## Patentansprüche

1. Enthaarungsartikel, der Folgendes umfasst:
(a) ein Substrat mit einer mittleren Stärke von 50 µm bis 15 µm (1); und
(b) eine wässrige Depilierzusammensetzung (2), die ein Keratinreduktionsmittel umfasst und mit dem Substrat in physischem Kontakt steht und vorzugsweise einen beschichteten oder imprägnierten Bereich des Substrats bildet;
wobei zumindest ein Abschnitt des Substrats transparent ist und wobei zumindest ein Abschnitt des beschichteten oder imprägnierten Bereichs des Substrats transparent ist.

2. Depilierartikel nach Anspruch 1, wobei der gesamte beschichtete oder imprägnierte Bereich des Substrats transparent ist.

3. Depilierartikel nach einem der vorstehenden Ansprüche, wobei die Depilierzusammensetzung transparent ist.

4. Depilierartikel nach einem der vorstehenden Ansprüche, wobei das Substrat, die Depilierzusammensetzung oder beide ferner mindestens eine Markierung zur Anleitung oder Hilfestellung für den Benutzer beim Halten, Positionieren, Auftragen, Verwenden oder Entfernen des Depilierartikels umfasst.

5. Depilierartikel nach einem der vorstehenden Ansprüche, wobei das Substrat nichtreflektierend ist.

6. Depilierartikel nach einem der vorstehenden Ansprüche, wobei das Substrat zumindest teilweise farblos, blau, grün, rosa oder lila ist, vorzugsweise farblos, blau oder grün und mehr bevorzugt farblos oder blau.

7. Depilierartikel nach einem der vorstehenden Ansprüche, wobei die Kombination aus dem Substrat und der Depilierzusammensetzung in zumindest einem Abschnitt des beschichteten oder imprägnierten Bereichs des Substrats transparent ist, wobei vorzugsweise die Kombination aus Substrat und Depilierzusammensetzung im gesamten beschichteten oder imprägnierten Bereich transparent ist.

8. Depilierartikel nach einem der vorstehenden Ansprüche, wobei die Depilierzusammensetzung eine mittlere Stärke von 0,01 mm bis 3 mm aufweist, vorzugsweise von 0,01 mm bis 1,5 mm, mehr bevorzugt von 0,05 mm bis 0,8 mm und noch mehr bevorzugt von 0,05 mm bis 0,5 mm.

9. Depilierartikel nach einem der vorstehenden Ansprüche, wobei das Substrat eine mittlere Stärke von 40 µm bis 16 µm und vorzugsweise von 30 µm bis 17 µm aufweist.

10. Depilierartikel nach einem der vorstehenden Ansprüche, wobei das Substrat elastisch ist, vorzugsweise eine Steifigkeit im Bereich von 5,00 g/cm bis 0,08 g/cm aufweist, mehr bevorzugt von 3,00 g/cm bis 0,08 g/cm, noch mehr bevorzugt von 1,80 g/cm bis 0,10 g/cm, noch mehr bevorzugt von 0,80 g/cm bis 0,15 g/cm und noch mehr bevorzugt von 0,60 g/cm bis 0,25 g/cm.

11. Depilierartikel nach einem der vorstehenden Ansprüche, wobei die Depilierzusammensetzung Wasser in einer Menge von mindestens 40 Gew.-% umfasst, vorzugsweise von 50 Gew.-% bis 98 Gew.-%, mehr bevorzugt von 60 Gew.-% bis 95 Gew.-% und noch mehr bevorzugt von 70 Gew.-% bis 90 Gew.-% der Depilierzusammensetzung.

12. Depilierartikel nach einem der vorstehenden Ansprüche, wobei die Depilierzusammensetzung eine Fließgrenze von 10 bis 2000 Pa aufweist, vorzugsweise von 30 bis 1200 Pa, mehr bevorzugt von 45 bis 500 Pa und noch mehr bevorzugt von 60 bis 300 Pa gemäß Messung in einem spannungsgesteuerten Amplitudentest bei einer Frequenz von 1 Hz und einer Temperatur von 25 °C.

13. Depilierartikel nach einem der vorstehenden Ansprüche, wobei das Substrat wasserundurchlässig ist, wobei vorzugsweise das Substrat ein wasserundurchlässiges Polymermaterial umfasst, mehr bevorzugt mindestens ein Polymerharz umfasst und noch mehr bevorzugt ein Polyolefin umfasst.

14. Verfahren zum Entfernen von Haar von der Haut, umfassend die folgenden Schritte:
(a) Auftragen eines Depilierartikels nach einem der vorstehenden Ansprüche auf die Hautoberfläche, vorzugsweise auf Säugetier- und mehr bevorzugt auf menschliche Haut,
(b) Belassen des Enthaarungsartikels in Kontakt mit der Haut für einen Zeitraum von mehr als 1 Minute, vorzugsweise 2 bis 10 Minuten, stärker bevorzugt 3 bis 8 Minuten
(c) Entfernen des Enthaarungsartikels von der Oberfläche der Haut und
(d) vorzugsweise Reiben, Kratzen oder Abwischen der Hautoberfläche in dem Bereich, in dem der Depilierartikel aufgetragen wurde

15. Enthaarungssatz, der Folgendes umfasst:
(a) Depilierartikel nach einem der Ansprüche 1 bis 13,
(b) mindestens eine einer Vorbehandlungs-Hautpflegezusammensetzung, einer Nachbehandlungs-Hautpflegezusammensetzung und/oder eines Werkzeugs, um die Entfernung von Haar und/oder der Enthaarungszusammensetzung nach Gebrauch zu unterstützen, und
(c) Verpackung für den Enthaarungssatz.

## Revendications

1. Article dépilatoire comprenant :
(a) un substrat avec une épaisseur moyenne allant de 50 µm à 15 µm (1) ; et
(b) une composition dépilatoire aqueuse (2) comprenant un agent réducteur de kératine et étant en contact physique avec le substrat, formant de préférence une région revêtue ou imprégnée du substrat ;
dans lequel au moins une partie du substrat est transparente et dans lequel au moins une partie de la région revêtue ou imprégnée du substrat est transparente.

2. Article dépilatoire selon la revendication 1, dans lequel l'intégralité de la région revêtue ou imprégnée du substrat est transparente.

3. Article dépilatoire selon l'une ou l'autre revendication précédente, dans lequel la composition dépilatoire est transparente.

4. Article dépilatoire selon l'une quelconque des revendications précédentes, dans lequel le substrat, la composition dépilatoire ou l'un et l'autre comprennent en outre au moins un marquage pour guider ou aider l'utilisateur à tenir, positionner, appliquer, utiliser ou retirer l'article dépilatoire.

5. Article dépilatoire selon l'une quelconque des revendications précédentes, dans lequel le substrat est non réfléchissant.

6. Article dépilatoire selon l'une quelconque des revendications précédentes, dans lequel le substrat est au moins partiellement incolore, bleu, vert, rose ou violet, de préférence incolore, bleu ou vert et plus préférablement incolore ou bleu.

7. Article dépilatoire selon l'une quelconque des revendications précédentes, dans lequel la combinaison du substrat et de la composition dépilatoire est transparente dans au moins une partie de la région revêtue ou imprégnée, de préférence dans lequel la combinaison du substrat et de la composition dépilatoire est transparente dans l'intégralité de la région revêtue ou imprégnée.

8. Article dépilatoire selon l'une quelconque des revendications précédentes, dans lequel la composition dépilatoire a une épaisseur moyenne allant 0,01 mm à 3 mm, de préférence de 0,01 mm à 1,5 mm, plus préférablement de 0,05 mm à 0,8 mm, et encore plus préférablement de 0,05 mm à 0,5 mm.

9. Article dépilatoire selon l'une quelconque des revendications précédentes, dans lequel le substrat a une épaisseur moyenne allant de 40 µm à 16 µm et de préférence de 30 µm à 17 µm.

10. Article dépilatoire selon l'une quelconque des revendications précédentes, dans lequel le substrat est souple, possède de préférence une rigidité dans l'intervalle allant de 5,00 g/cm à 0,08 g/cm, plus préférablement de 3,00 g/cm à 0,08 g/cm, encore plus préférablement de 1,80 g/cm à 0,10 g/cm, même plus préférablement encore de 0,80 g/cm à 0,15 g/cm et encore plus préférablement de 0,60 g/cm à 0,25 g/cm.

11. Article dépilatoire selon l'une quelconque des revendications précédentes, dans lequel la composition dépilatoire comprend de l'eau en une quantité d'au moins 40 %, de préférence de 50 % à 98 %, plus préférablement de 60 % à 95 % et encore plus préférablement de 70 % à 90 % en poids de la composition dépilatoire.

12. Article dépilatoire selon l'une quelconque des revendications précédentes, dans lequel la composition dépilatoire a une limite de résistance élastique allant de 10 à 2000 Pa, de préférence de 30 à 1200 Pa, plus préférablement de 45 à 500 Pa et encore plus préférablement de 60 à 300 Pa lorsqu'on mesure par l'intermédiaire d'un balayage à amplitude contrôlée en contrainte à une fréquence de 1 Hz et une température de 25 °C.

13. Article dépilatoire selon l'une quelconque des revendications précédentes, dans lequel le substrat est imperméable à l'eau, de préférence dans lequel le substrat comprend un matériau polymère imperméable à l'eau, plus préférablement comprend au moins une résine polymère, et encore plus préférablement comprend une polyoléfine.

14. Procédé d'élimination de poils de la peau, comprenant les étapes consistant à :
(a) appliquer un article dépilatoire selon l'une quelconque des revendications précédentes sur la surface de la peau, de préférence la peau mammalienne et plus préférablement la peau humaine,
(b) laisser ledit article dépilatoire en contact avec la peau pendant une période supérieure à 1 minute, de préférence 2 à 10 minutes, plus préférablement 3 à 8 minutes
(c) retirer ledit article dépilatoire de la surface de la peau, et
(d) de préférence frotter, racler ou essuyer la surface de la peau dans la zone sur laquelle l'article dépilatoire a été appliqué.

15. Trousse dépilatoire, comprenant :
(a) un article dépilatoire selon l'une quelconque des revendications 1 à 13,
(b) au moins un élément parmi une composition de soin de la peau de prétraitement, une composition de soin de la peau de post-traitement et/ou un outil pour faciliter le retrait des poils et/ou de la composition dépilatoire après utilisation, et
(c) un conditionnement pour ladite trousse dépilatoire.
